# EUROPEAN PATENT SPECIFICATION

(11) **EP 1 628 700 B1**
(45) Date of publication and mention of the grant of the patent: **21.12.2011**
(21) Application number: 04753425.0
(22) Date of filing: 25.05.2004
(51) Int. Cl.: A61M 25/00

(54) **HIGH PRESSURE CATHETER AND METHODS FOR MANUFACTURING THE SAME**
HOCHDRUCKKATHETER UND HERSTELLUNGSVERFAHREN DAFÜR
CATHETER A HAUTE PRESSION ET PROCEDES DE FABRICATION DE CE CATHETER

(30) Priority: 28.05.2003 US 473683 P
(43) Date of publication of application: 01.03.2006
(73) Proprietor: C.R. BARD, INC., Murray Hill New Jersey 07974 (US)
(72) Inventor: KING, Eric, West Jordan, UT 84084 (US); WORTLEY, Ron, Salt Lake City, UT 84121 (US); DIAMOND, Jordan, P., Salt Lake City, UT 84106 (US)
(74) Representative: Siegert, Georg
(86) International application number: PCT/US2004/016595
(87) International publication number: WO 2004/105850

(56) References cited:
- WO-A-97/22374
- WO-A-99/16498
- US-A- 4 588 402
- US-A- 4 969 879
- US-A- 5 160 325
- US-A- 5 718 678
- US-A- 5 776 117
- US-A- 5 797 869
- US-A- 5 961 485

## Description

### CROSS-REFERENCE TO RELATED APPLICATIONS

This application claims the benefit of United States provisional application Serial No. 60/473,683, filed May 28, 2003.

### FIELD OF THE INVENTION

The invention generally relates to medical devices and methods for manufacturing such medical devices. In particular, the invention relates to catheters, methods for making such catheters, and methods for using such catheters. More particularly, the invention relates to power injection catheters, methods for manufacturing such catheters to address the high-pressure requirements and applications, and methods for using such catheters for high-pressure vascular access.

### BACKGROUND OF THE INVENTION

Catheters and their use as medical devices are well known in the art. Some medical procedures, such as CT Scans, typically require rapid infusion of contrast media into the vascular system. These types of procedures are currently accomplished by accessing the vascular system with a needle and cannula that is connected to a power injection machine. The injection pressure of the machine is set to a clinically predetermined limit that is relatively high. When activated, the machine rapidly injects the media into the vascular system of the patient at a flow rate that will not exceed the predetermined pressure limit. The wide variation in the viscosity of different contrast media or medications, coupled with rapid flow rate requirements, can often result in a wide range of pressures.

A large number of patients that require the procedure described above already have a typical implanted catheter that is being used for other medical procedures. Typical catheter designs usually contain three major components: (1) the catheter shaft, (2) an extension tube assembly, and (3) a bifurcation in the case of multi lumen catheters or a hub in the case of a single lumen catheter. As used herein, the term "catheter" may refer to a device comprising each of said three major components. The catheter shaft is the portion of the catheter that is inserted into the vascular system. The extension tube assembly is the portion of the catheter that provides vascular access from outside of the body. The bifurcation or hub is the portion of the catheter that connects to the proximal end of the catheter shaft, connecting the catheter shaft to the extension tube assembly. The bifurcation or hub usually contains geometry required to secure the catheter to the body such as suture wings. The bifurcation or hub also provides a location to print instructions or logos on the catheter surface.

Typical catheters are usually designed and manufactured with flexible materials that are compliant. Using flexible materials increases patient comfort, especially when securing the catheter to the body, and also reduces trauma to the vascular system. As well, the catheter shaft and extension tube assembly are typically made of thin walled tubing. The strength of the tubing, especially for high pressure applications, can be increased (within certain limits) by increasing the durometer of the raw materials while retaining most of the flexibility and comfort of the assembly. Such an increase in strength by increasing the durometer of the raw material, however, is not possible with respect to the bifurcation and hub due to the relatively thicker geometry thereof. More particularly, due to the thicker geometry, any increase in the durometer of the raw material would greatly increase the stiffness of the bifurcation and hub, which increased stiffness would negatively affect patient comfort and the ability to attach the bifurcation and hub to the patient's body.

Thus, improving the pressure resistance of a typical catheter assembly, such as a venous access catheter assembly that can usually only withstand pressures of up to approximately 150 psi (1.03 N/mm²) has proved difficult due to the inability to provide a bifurcation and hub having a pressure resistance that matches that of the tubing. Accordingly, typical catheters do not exhibit the properties that are needed for the high-pressure (i.e., power injection) medical procedures described above, meaning that the patient must undergo more invasive procedures, such as repeated vascular access (i.e., needle sticks).

### BRIEF SUMMARY OF THE INVENTION

The present invention provides high pressure catheters, methods for manufacturing such catheters to address high-pressure requirements and applications, and methods for using such catheters for high-pressure vascular access. The high pressure catheters can be produced by maintaining the typical soft flexible exterior in the bifurcation, hub, and/or suture wings, while providing a more rigid high-pressure connection interior. The stiffer internal geometry transforms the typical catheter into a high-pressure catheter and allows a single implanted catheter to perform both standard and high-pressure functions, thereby reducing the number of vascular access procedures required during a course of therapy.

A catheter according to the present invention comprises a catheter shaft, an extension tube assembly, a single lumen, barbed inner hub comprising a rigid hard material, connecting said catheter shaft to said extension tube assembly, wherein said catheter shaft is in fluid communication with said extension tube assembly, and an outer hub comprising a soft flexible material molded around said single lumen, barbed inner hub.

A method for making a catheter comprising a catheter shaft and an extension tube assembly, comprises the steps of placing said catheter shaft and said extension tube assembly into a first mold, wherein a proximal end of said catheter shaft is positioned adjacent a distal end of said extension tube assembly within a first mold cavity, insert molding an inner hub comprising a rigid hard material over said proximal end of said catheter shaft and said distal end of said extension tube assembly, placing the combination of said inner hub, catheter shaft and extension tube assembly into a second mold, wherein said inner hub is placed within a second mold cavity, said inner hub being supported and centered therewithin, and insert molding an outer hub comprising a soft flexible material over said inner hub.

The present invention provides a method for making a catheter comprising a catheter shaft and an extension tube assembly, wherein said catheter shaft and said extension tube assembly each comprise a single lumen, which comprises the steps of inserting a portion of a single lumen, barbed inner hub having a throughgoing lumen into a proximal end of said catheter shaft lumen and a distal end of said extension tube assembly lumen such that said single lumen, barbed inner hub is substantially contained within said catheter shaft and said extension tube assembly, wherein said catheter shaft is in fluid communication with said extension tube assembly, placing the combination of said single lumen, barbed inner hub, catheter shaft and extension tube assembly into a mold, wherein said single lumen, barbed inner hub is positioned within a mold cavity, and insert molding an outer hub comprising a soft flexible material over said catheter shaft and extension tube assembly.

These and other embodiments, features and advantages of the present invention will become more apparent to those skilled in the art when taken with reference to the following more detailed description of the invention in conjunction with the accompanying drawings that are first briefly described.

### BRIEF DESCRIPTION OF THE DRAWINGS

FIG. 1 is a side perspective view of a rigid single lumen inner hub, insert molded over a catheter shaft and extension tube assembly, according to one aspect of the present disclosure.

FIG. 2 is a side perspective view of a soft flexible single lumen outer hub, insert molded over the rigid inner hub of FIG. 1;

FIG. 3 is a side perspective view of a rigid dual lumen inner hub, insert molded over a catheter shaft and extension tube assembly, according to one aspect of the present disclosure.

FIG. 4 is a side perspective view of a soft flexible dual lumen outer hub, insert molded over the rigid inner hub of FIG. 3.

FIG. 5 is a side perspective view of a single lumen rigid inner hub, according to an aspect of the present invention.

FIG. 6 is a side perspective exploded view of a single lumen catheter shaft, extension tube assembly and rigid inner hub of FIG. 5.

FIG. 7 is a side perspective view of the components of FIG. 6 assembled.

FIG. 8 is a side perspective view of a soft flexible single lumen outer hub, insert molded over the rigid inner hub of FIG. 7.

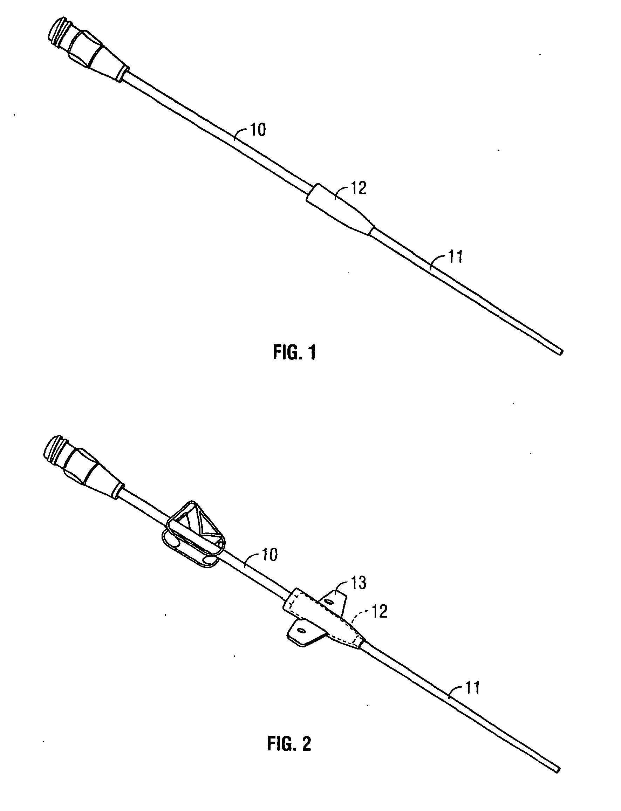

FIGS. 1-8 illustrate specific aspects of the disclosure and are a part of the specification. Together with the following description, these figures demonstrate and explain the principles of the disclosure.

### DETAILED DESCRIPTION OF THE INVENTION

The following description provides specific details in order to provide a thorough understanding of the invention. The skilled artisan, however, would understand that the invention can be practiced without employing these specific details. Indeed, the invention can be practiced by modifying the illustrated method and resulting product and can be used in conjunction with apparatus and techniques conventionally used in the industry. For example, the invention is described for a single or dual lumen catheter. The invention, however, could easily be adapted for three or four (or even more) lumen catheters. In another example, the manufacturing process of the invention is described primarily in terms of insert molding or solvent bonding relating to plastic as the base material in the context of catheter applications. However, the invention could be used with manufacturing processes that require snap fits using metal components as the rigid medical inner structure of the bifurcation or hub.

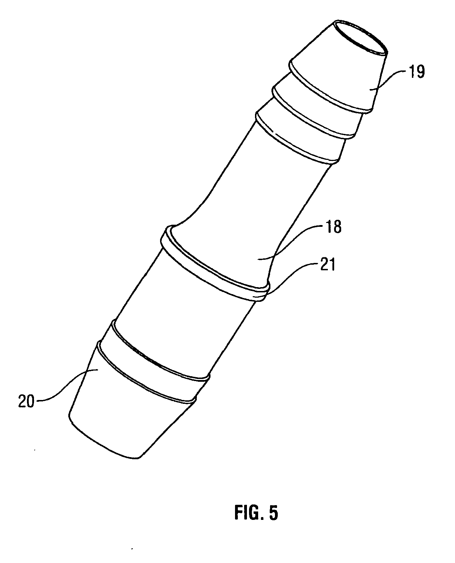

The invention provides a catheter capable of high-pressure applications while maintaining the look and feel of standard catheter devices: The high pressure catheter of the invention maintains the typical soft flexible exterior in the bifurcation, hub, and/or suture wings while providing a more rigid high-pressure connection interior. One example of such a catheter is illustrated in FIGS. 5-8. It should be appreciated in the following examples of the disclosure that the rigid inner hub (*i.e*., inner hub 12, 16, 18) may be formed from a material such as medical grade polyurethane (*e.g*., Tecoflex^{®}, Carbothane^{®}, Pelathane^{®}, Chronoflex^{®}, Tecoplast^{®}, etc.) having a hardness in the range of approximately 90 Shore A durometer to 80 Shore D durometer, with the currently preferred polyurethane being Tecoflex^{®} having a hardness in the range of approximately 70-75 Shore D durometer. The rigid inner hub may also be formed from other materials, such as metal (*e.g* , stainless steel, titanium, etc.), polyvinylchloride, nylon, polyester, and castable epoxy, provided the materials are sufficiently hard to withstand pressures present in typical high pressure catheter applications. The outer hub, on the other hand, may be formed from a much softer, flexible material, which is suitable for contacting the skin of a patient, such as medical grade polyurethane having a hardness rating in the range of approximately 35-80 Shore A durometer. The outer hub may also be formed from silicone or other materials discussed herein, provided that the material utilized is sufficiently soft to provide flexibility and patient comfort.

One aspect of the disclosure is illustrated in FIGS. 1-2. FIG. 1 depicts a single lumen rigid inner hub 12 that forms a rigid high pressure connection between the fluid paths of catheter shaft 11 and extension tube assembly 10. Inner hub 12 may be insert molded directly over both the catheter shaft 11 and the extension tube assembly 10, or may be separately molded as a pre-formed insert and attached to the catheter shaft 11 and extension tube assembly 10 manually (*i.e.,* catheter shaft 11 and extension tube assembly 10 are respectively inserted into opposite ends of the inner hub 12). In the case of a separately molded inner hub 12, variations include having through holes with lead-in tapers, the use of adhesives, and or having barbs or other fittings that would ensure a tight fit of the catheter shaft 11 and extension tube assembly 10 within the inner hub 12. FIG. 2 depicts a soft pliable outer hub 13 that contains flexible suture wings substantially similar to those found on typical catheters. In one method of manufacture, the outer hub 13 can be insert molded over the inner hub 12, such that the geometry of the inner hub 12 is supported during the insert molding process by the outer hub 13 mold cavity, thereby keeping the inner hub 12 located in the center of the molded assembly.

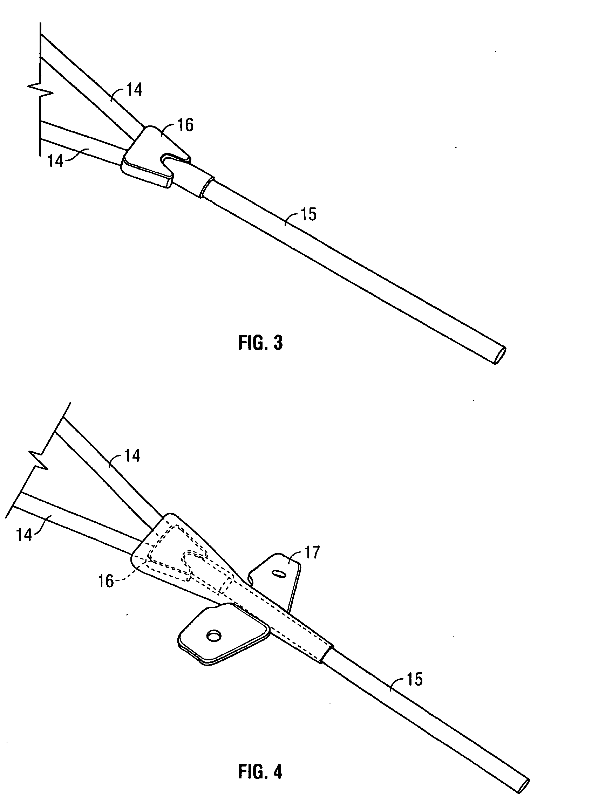

Another aspect of the disclosure is illustrated in FIGS. 3-4. FIG. 3 depicts a dual lumen rigid inner hub (or inner connector) 16 that forms a rigid high pressure connection between the fluid paths of dual lumen catheter shaft 15 and the extension tube assembly 14. Like inner hub 12, inner hub 16 may be insert molded directly over both the dual lumen catheter shaft 15 and the extension tube assembly 14, or may be separately molded as a pre-formed insert and manually attached to the dual lumen catheter shaft 15 and the extension tube assembly 14 in the same manner as described above in connection with inner hub 12. FIG. 4 depicts a soft pliable dual lumen outer hub 17 containing flexible suture wings substantially similar to those found on typical catheters. The outer hub 17 can be insert molded over the inner hub 16. As with the single lumen embodiment described above, the geometry of the inner hub 16 can be supported during the insert molding process by the outer hub 17 mold cavity, thereby keeping the inner hub 16 located in the center of the molded assembly.

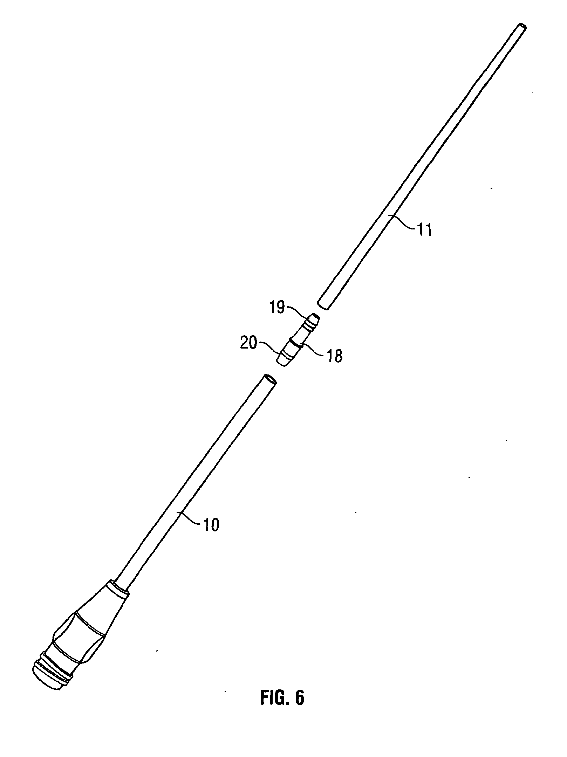

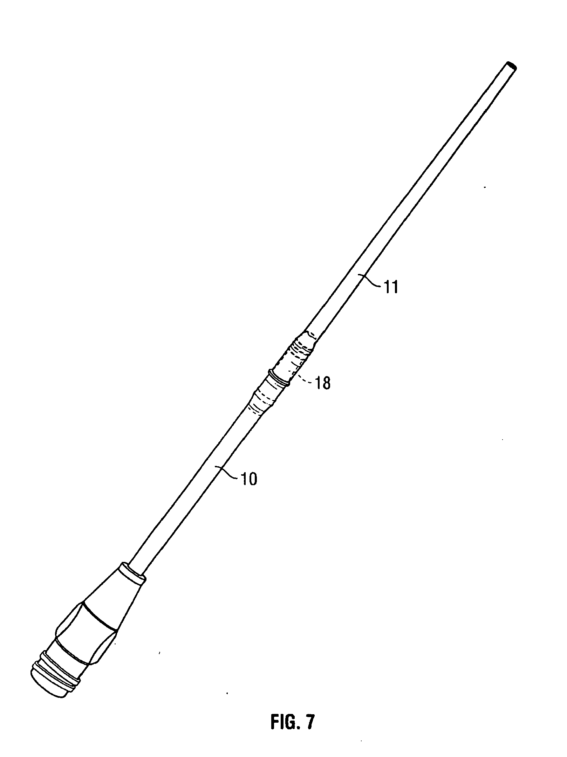

An aspect of the invention is illustrated in FIGS. 5-8, depicting a method of design and manufacture of a high-pressure inner hub according to the present invention. As illustrated in Fig.5, an inner hub 18 contains distal barbs 19 for retention of the catheter shaft 11 and proximal barbs 20 for retention of the extension tube assembly 10, as well as a ring configuration 21 around a central portion thereof. FIG. 6 shows an exploded view of the inner hub 18 in relationship to the catheter shaft 11 and the extension tube assembly 10. FIG. 7 depicts the inner hub 18 with the catheter shaft 11 pressed over the distal retention barbs 19, and extension tube assembly 10 pressed over the proximal retention barbs 20, such that the proximal end of the catheter shaft 11 and the distal end of the extension tube assembly 10 abut the ring configuration 21. Such a configuration forms a rigid high-pressure connection between the fluid paths of the catheter shaft 11 and extension tube assembly 10.

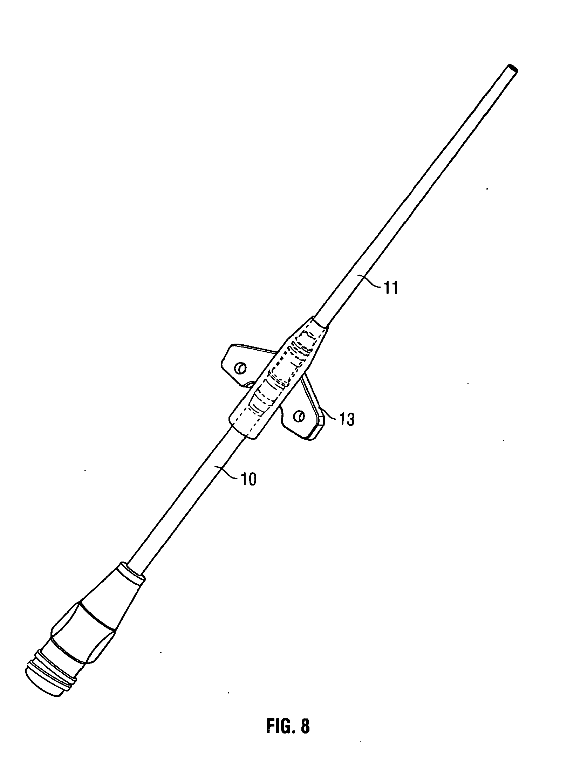

In an alternate embodiment, a compression sleeve could be placed over the inner hub 18, following insertion into the catheter shaft 11 and the extension tube assembly 10, such that the compression sleeve covered the ring configuration 21 and portions of the catheter shaft 11 and the extension tube assembly 10. The compression sleeve could then be crimped onto the assembly to provide a smooth outer surface for the transition between the catheter shaft 11 and the extension tube assembly 10. FIG. 8 depicts a soft pliable single lumen outer hub 17 containing flexible suture wings substantially similar to those found on typical catheters. The outer hub 17 is insert molded over the inner hub 18 similar to that described above in connection with FIG. 2.

As described above, the high pressure catheters are made with a soft flexible exterior in the connector/hub while providing a more rigid high-pressure interior. Any manufacturing process known in the art that can provide this soft flexible exterior and rigid, high-pressure interior can be used to make the hub of the disclosure. In one aspect of the disclosure the inner hub is made by inserting a ridged core pin between the extension tube assembly and the catheter shaft. As with standard insert molding practice, use of a core pin keeps the fluid path between the extension tube assembly and catheter shaft open during the molding process. The extension tube assembly, catheter shaft and core pin combination are loaded into a mold, which is machined to create a cavity having the desired shape and geometry of the inner hub. Molten high durometer (ridged) plastic is then injected into the mold cavity, encapsulating the extension tube assembly and catheter shaft. The core pin is then removed, leaving a ridged high-pressure connection between the extension tube assembly and catheter shaft. The outer hub is produced in a similar procedure. A ridged core pin is inserted into the ridged high-pressure connection described above and the combination is loaded into a mold, which is machined to create a cavity with the desired shape and geometry of the outer hub. Molten low durometer (soft) plastic is then injected into the mold cavity, encapsulating the ridged high-pressure connection. The core pin is then removed, leaving a soft flexible outer hub over the rigid high-pressure connection between the extension tube assembly and catheter shaft.

Using the hub of the invention, a typical catheter can be used in both standard pressure and high-pressure medical procedures. Thus, for example, a single catheter can be used for both procedures, saving equipment cost (using a single catheter in place of two or more catheters), physician time (needing only a single needle prick), and safer, less intrusive procedures (requiring only a single vascular access site).

In addition to any previously indicated variation, numerous other modifications and alternative arrangements may be devised by those skilled in the art without departing from the scope of the invention and appended claims are intended to cover such modifications and arrangements. Thus, while the invention has been described above with particularity and detail in connection with what is presently deemed to be the most practical and preferred aspects of the invention, it will be apparent to those of ordinary skill in the art that numerous modifications, including but not limited to, form, function, manner of operations and use may be made without departing form the principles and concepts set forth herein.

## Claims

1. A catheter, comprising:
a catheter shaft (11);
an extension tube assembly (10);
**characterised by**
a single lumen, barbed (19, 20) inner hub (18) comprising a rigid hard material, connecting said catheter shaft to said extension tube assembly, wherein said catheter shaft is in fluid communication with said extension tube assembly; and
an outer hub (17) comprising a soft flexible material, molded around said single lumen, barbed inner hub.

2. The catheter according to claim 1, wherein said catheter shaft and said extension tube assembly each comprise a single lumen.

3. The catheter according to claim 2, wherein a portion of said inner hub is inserted into a proximal end of said catheter shaft lumen and a distal end of said extension tube assembly lumen.

4. The catheter according to claim 3, wherein said inner hub is substantially contained within said catheter shaft and said extension tube assembly.

5. The catheter according to claim 3, wherein said inner hub comprises distal and proximal barbs.

6. The catheter according to claim 5, wherein said inner hub further comprises a central ring.

7. The catheter according to claim 2, wherein said inner hub comprises openings configured for receiving said catheter shaft and said extension tube assembly.

8. The catheter according to claim 7, wherein a lumen of said inner hub comprises tapered portions adjacent to said openings.

9. The catheter according to claim 1, wherein said catheter shaft comprises an outer wall enclosing at least two lumens therein, and wherein said extension tube assembly comprises at least two separate tubes in respective fluid communication with each of said at least two lumens.

10. The catheter according to claim 1, wherein said rigid hard material comprises a material selected from the group consisting of medical grade polyurethane, metal, polyvinylchloride, nylon, polyester, and castable epoxy.

11. The catheter according to claim 10, wherein said rigid hard material comprises medical grade polyurethane having a hardness in the range of approximately 90 Shore A durometer to 80 Shore D durometer.

12. The catheter according to claim 11, wherein said rigid hard material comprises Tecoflex® having a hardness in the range of approximately 70-75 Shore D durometer.

13. The catheter according to claim 1, wherein said soft flexible material comprises a material selected from the group consisting of medical grade polyurethane, silicone, polyvinylchloride, nylon, polyester, and castable epoxy.

14. The catheter according to claim 13, wherein said soft flexible material comprises silicone having a hardness in the range of approximately 35-80 Shore A durometer.

15. The catheter according to claim 1, wherein said rigid hard material is adapted to accommodate pressures higher than approximately 150 psi.

16. The catheter according to claim 1, wherein said outer hub comprises a pair of suture wings.

17. A method for making a catheter comprising a catheter shaft and an extension tube assembly, wherein said catheter shaft and said extension tube assembly each comprise a single lumen, comprising the steps of:
inserting a portion of a single lumen, barbed inner hub having a throughgoing lumen into a proximal end of said catheter shaft lumen and a distal end of said extension tube assembly lumen such that said single lumen, barbed inner hub is substantially contained within said catheter shaft and said extension tube assembly, wherein said catheter shaft is in fluid communication with said extension tube assembly;
placing the combination of said single lumen, barbed inner hub, catheter shaft and extension tube assembly into a mold, wherein said single lumen, barbed inner hub is positioned within a mold cavity; and
insert molding an outer hub comprising a soft flexible material over said catheter shaft and extension tube assembly.

## Patentansprüche

1. Katheter mit:
einem Katheterschaft (11),
einem Verlängerungsrohraufbau (10),
**gekennzeichnet durch**
einen einlumigen mit Widerhaken (19, 20) versehenen inneren Knotenpunkt (18), der ein steifes hartes Material aufweist und den Katheterschaft mit dem Verlängerungsrohraufbau verbindet, wobei der Katheterschaft mit dem Verlängerungsrohraufbau in Fluidverbindung steht; und
einen äußeren Knotenpunkt (17) mit einem weichen flexiblen Material, das um den einlumigen mit Widerhaken versehenen inneren Knotenpunkt umspritzt ist.

2. Katheter gemäß Anspruch 1, bei dem der Katheterschaft und der Verlängerungsrohraufbau jeweils ein einzelnes Lumen aufweisen.

3. Katheter gemäß Anspruch 2, bei dem ein Abschnitt des inneren Knotenpunkts in ein proximales Ende des Katheterschaftlumens und einem distalen Ende des Verlängerungsrohraufbaulumens eingeführt wird.

4. Katheter gemäß Anspruch 3, bei dem der innere Knotenpunkt im Wesentlichen innerhalb des Katheterschafts und des Verlängerungsrohraufbaus enthalten ist.

5. Katheter gemäß Anspruch 3, bei dem der innere Knotenpunkt distale und proximale Widerhaken aufweist.

6. Katheter gemäß Anspruch 5, bei dem der innere Knotenpunkt des Weiteren einen mittigen Ring aufweist.

7. Katheter gemäß Anspruch 2, bei dem der innere Knotenpunkt Öffnungen aufweist, die zum Aufnehmen des Katheterschafts und des Verlängerungsrohraufbaus eingerichtet sind.

8. Katheter gemäß Anspruch 7, bei dem ein Lumen des inneren Knotenpunkts zu den Öffnungen angrenzende sich verjüngende Abschnitte aufweist.

9. Katheter gemäß Anspruch 1, bei dem der Katheterschaft eine Außenwand aufweist, die in sich mindestens zwei Lumen umschließt und wobei der Verlängerungsrohraufbau mindestens zwei getrennte Röhren aufweist, die jeweils mit jedem der mindestens zwei Lumen in Fluidverbindung stehen.

10. Katheter gemäß Anspruch 1, bei dem das steife harte Material ein Material aufweist, das aus der aus medizinischem Polyurethan, Metall, Polyvinylchlorid, Nylon, Polyester und gießbarem Epoxyd bestehenden Gruppe ausgewählt ist.

11. Katheter gemäß Anspruch 10, bei dem das steife harte Material medizinisches Polyurethan mit einer Härte im Bereich von näherungsweise 90 Shore A Durometer bis 80 Shore D Durometer aufweist.

12. Katheter gemäß Anspruch 11, bei dem das steife harte Material Tecoflex^{®} aufweist, das eine Härte im Bereich von näherungsweise 70-75 Shore D Durometer aufweist.

13. Katheter gemäß Anspruch 1, bei dem das weiche flexible Material ein Material aufweist, das aus der aus medizinischem Polyurethan, Silikon, Polyvinylchlorid, Nylon, Polyester und gießbarem Epoxyd bestehenden Gruppe ausgewählt ist.

14. Katheter gemäß Anspruch 13, bei dem das weiche flexible Material Silicon aufweist, das eine Härte in dem Bereich von näherungsweise 35-80 Shore A Durometer aufweist.

15. Katheter gemäß Anspruch 1, bei dem das steife harte Material eingestellt ist, um Drücke höher als in etwa 150 psi aufzunehmen.

16. Katheter gemäß Anspruch 1, bei dem der äußere Knotenpunkt ein paar Nahtflügel aufweist.

17. Verfahren zur Herstellung eines Katheters mit einem Katheterschaft und einem Verlängerungsrohraufbau, wobei der Katheterschaft und der Verlängerungsrohraufbau jeweils ein einzelnes Lumen aufweisen und das die Schritte aufweist:
Einführen eines Abschnitts eines einlumigen mit Widerhaken versehenen inneren Knotenpunkts mit einem durchgehenden Lumen in das proximale Ende des Katheterschaftlumens und ein distales Ende des Verlängerungsrohraufbaulumens, sodass der einlumige mit Widerhaken versehene innere Knotenpunkt im Wesentlichen in dem Katheterschaft und dem Verlängerungsrohraufbau aufgenommen ist, wobei der Katheterschaft mit dem Verlängerungsrohraufbau in Fluidverbindung steht;
Platzieren der Kombination des einlumigen mit Widerhaken versehenen Knotenpunkts, Katheterschafts und Verlängerungsrohraufbaus in einer Form, wobei der einlumige mit Widerhaken versehene innere Knotenpunkt innerhalb einer Formvertiefung positioniert wird; und
Spritzgießen eines äußeren Knotenpunkts, der ein weiches flexibles Material aufweist, über den Katheterschaft und den Verlängerungsrohraufbau.

## Revendications

1. Cathéter, comprenant :
une tige (11) de cathéter ;
un ensemble (10) de tubes de prolongement ; **caractérisé par**
un raccord interne (18) à crans (19, 20), mono-lumière comprenant un matériau dur et rigide, reliant ladite tige de cathéter audit ensemble de tubes de prolongement, où ladite tige de cathéter est en communication fluidique avec ledit ensemble de tubes de prolongement ; et
un raccord externe (17) comprenant un matériau souple et flexible, moulé autour dudit raccord interne à crans, mono-lumière.

2. Cathéter selon la revendication 1, dans lequel ladite tige de cathéter et ledit ensemble de tubes de prolongement comprennent chacun une seule lumière.

3. Cathéter selon la revendication 2, dans lequel une partie dudit raccord interne est insérée dans une extrémité proximale de ladite lumière de tige de cathéter et dans une extrémité distale de ladite lumière d'ensemble de tubes de prolongement.

4. Cathéter selon la revendication 3, dans lequel ledit raccord interne est essentiellement contenu dans ladite tige de cathéter et dans ledit ensemble de tubes de prolongement.

5. Cathéter selon la revendication 3, dans lequel ledit raccord interne comprend des crans distal et proximal.

6. Cathéter selon la revendication 5, dans lequel ledit raccord interne comprend en outre un anneau central.

7. Cathéter selon la revendication 2, dans lequel ledit raccord interne comprend des ouvertures configurées pour recevoir ladite tige de cathéter et ledit ensemble de tubes de prolongement.

8. Cathéter selon la revendication 7, dans lequel une lumière dudit raccord interne comprend des parties coniques adjacentes auxdites ouvertures.

9. Cathéter selon la revendication 1, dans lequel ladite tige de cathéter comprend une paroi externe y renfermant au moins deux lumières, et dans lequel ledit ensemble de tubes de prolongement comprend au moins deux tubes séparés en communication fluidique respective avec chacune desdites au moins deux lumières.

10. Cathéter selon la revendication 1, dans lequel ledit matériau dur et rigide comprend un matériau choisi parmi le groupe constitué de polyuréthane de qualité médicale, de métal, de chlorure de polyvinyle, de nylon, de polyester et d'époxy à couler.

11. Cathéter selon la revendication 10, dans lequel ledit matériau dur et rigide comprend du polyuréthane de qualité médicale ayant une dureté dans la plage d'approximativement 90 au duromètre Shore A à 80 au duromètre Shore D.

12. Cathéter selon la revendication 11, dans lequel ledit matériau dur et rigide comprend Tecoflex ® ayant une dureté dans la plage d'approximativement 70 à 75 au duromètre Shore D.

13. Cathéter selon la revendication 1, dans lequel ledit matériau souple et flexible comprend un matériau choisi parmi le groupe constitué de polyuréthane de qualité médicale, de silicone, de chlorure de polyvinyle, de nylon, de polyester et d'époxy à couler.

14. Cathéter selon la revendication 13, dans lequel ledit matériau souple et flexible comprend du silicone ayant une dureté dans la plage d'approximativement 35-80 au duromètre Shore A.

15. Cathéter selon la revendication 1, dans lequel ledit matériau dur et rigide est adapté pour supporter des pressions supérieures à environ 150 psi.

16. Cathéter selon la revendication 1, dans lequel ledit raccord externe comprend une paire d'ailes de suture.

17. Procédé de fabrication d'un cathéter comprenant une tige de cathéter et un ensemble de tubes de prolongement, où ladite tige de cathéter et ledit ensemble de tubes de prolongement comprennent chacun une seule lumière, comprenant les étapes qui consistent :
à insérer une partie d'un raccord interne à crans, mono-lumière ayant une lumière traversante dans une extrémité proximale de ladite lumière de tige de cathéter et dans une extrémité distale de ladite lumière d'ensemble de tubes de prolongement de sorte que ledit raccord interne à crans, mono-lumière soit essentiellement contenu dans ladite tige de cathéter et dans ledit ensemble de tubes de prolongement, où ladite tige de cathéter est en communication fluidique avec ledit ensemble de tubes de prolongement ;
à placer la combinaison desdits raccord interne à crans, mono-lumière, tige de cathéter et ensemble de tubes de prolongement dans un moule, où ledit raccord interne à crans, mono-lumière est positionné à l'intérieur d'une cavité du moule ; et
à mouler par insertion un raccord externe comprenant un matériau souple et flexible sur lesdits tige de cathéter et ensemble de tubes de prolongement.
